# EUROPEAN PATENT SPECIFICATION

(11) **EP 2 050 817 B1**
(45) Date of publication and mention of the grant of the patent: **01.05.2013**
(21) Application number: 07792487.6
(22) Date of filing: 14.08.2007
(51) Int. Cl.: C12Q 1/68, C12N 15/00, C12N 15/09, G01N 21/78

(54) **NUCLEIC ACID STRAND USEFUL IN DETECTING SUBSTANCE AND METHOD THEREOF**
ZUM NACHWEIS EINER SUBSTANZ GEEIGNETER NUKLEINSÄURESTRANG UND VERFAHREN DAFÜR
BRIN D'ACIDE NUCLEIQUE UTILE POUR DETECTER UNE SUBSTANCE ET PROCEDE DE DETECTION CORRESPONDANT

(30) Priority: 14.08.2006 JP 2006221228; 03.08.2007 JP 2007203079
(43) Date of publication of application: 22.04.2009
(73) Proprietor: Sony Corporation, Tokyo 108-0075 (JP)
(72) Inventor: NAKAGAWA, Kazuhiro, Minato-ku Tokyo 108-0075 (JP)
(74) Representative: Müller - Hoffmann & Partner
(86) International application number: PCT/JP2007/065844
(87) International publication number: WO 2008/020589

(56) References cited:
- WO-A1-98/04738
- WO-A2-00/11446
- JP-A- 02 503 515
- JP-A- 06 153 997
- JP-A- 2005 192 418
- US-A1- 2004 101 893
- ADLER M ET AL: "A real-time immuno-PCR assay for routine ultrasensitive quantification of proteins" BIOCHEMICAL AND BIOPHYSICAL RESEARCH COMMUNICATIONS, ACADEMIC PRESS INC. ORLANDO, FL, US LNKD- DOI:10.1016/S0006-291X(03)01364-0, vol. 308, no. 2, 22 August 2003 (2003-08-22), pages 240-250, XP004442967 ISSN: 0006-291X
- TOMBELLI S ET AL: "Analytical applications of aptamers" BIOSENSORS AND BIOELECTRONICS, ELSEVIER BV, NL LNKD- DOI:10.1016/J.BIOS.2004.11.006, vol. 20, no. 12, 15 June 2005 (2005-06-15) , pages 2424-2434, XP004861158 ISSN: 0956-5663
- CAIRNS M.J. ET AL.: 'Homogeneous real-time detection and quantification of nucleic acid amplification using restriction enzyme digestion' BIOCHEM. BIOPHYS. RES. COMMUN. vol. 318, no. 3, 2004, pages 684 - 690, XP004508488

## Description

### Technical Field

The present invention relates to a nucleic acid strand useful in detecting a substance, etc., and a technique related to the nucleic acid strand. More particularly, the invention relates to a nucleic acid strand which has an enzyme cleavage site in a nucleotide sequence region between a fluorescent substance and a quencher substance, and a method using the nucleic acid strand.

### Background Art

Techniques for detecting the presence of a substance or reaction thereof, etc. by detecting fluorescence emitted from the substance are known, and they are widely applied to various sensor techniques and the like.

For example, a technique in which the presence of a substance present in a reaction field is confirmed by introducing a probe substance capable of specifically interacting with the substance into the reaction field and determining whether or not fluorescence of the probe substance which have been fluorescently labeled in advance can be detected in the reaction field is known. For example, a technique in which a fluorescently labeled nucleic acid strand is introduced for a nucleic acid strand immobilized on a substrate (chip) surface and the presence or absence of hybridization between both nucleic acid strands is detected by fluorescence detection is a commonly used technique for a DNA chip.

Further, so-called "intercalator", a substance that emits fluorescence by specifically binding to the complementary binding site when single-stranded nucleic acids present in a reaction field are hybridized with each other, is also known. Such an intercalator has an advantage that a procedure of labeling a nucleic acid strand with a fluorescent substance in advance can be omitted, etc.

"FRET (fluorescence resonance energy transfer)" refers to a phenomenon or a principle that a fluorescent substance (acceptor) which receives resonance excitation energy released from an excited fluorescent substance (donor) is excited to emit fluorescence. In order to cause this "FRET", it is required that the fluorescence spectra of the donor and the acceptor overlap each other and both substances be located within a fixed range or so or in a proper positional relationship. A technique in which the presence or absence of an interaction between a substance labeled with a fluorescent substance serving as a donor and a substance labeled with a fluorescent substance serving as an acceptor is detected using this "FRET" is known (see, for example, JP-A-2004-065262 and JP-A-2005-207823).

Subsequently, "bioluminescence resonance energy transfer (BRET)" is a biophysical method capable of directly detecting a protein-protein interaction. This "BRET" is originally a process observed in marine organisms such as Aequorea victoria and Renilla reniformis, and release of energy from an acceptor protein accompanying the transfer of excitation energy from a donor protein which is caused when the donor protein and the acceptor protein come close to each other can be detected by fluorescence detection. Therefore, BRET can be a technique useful in detecting an interaction between proteins.

A technique generally employed when an infinitesimal substance present in a reaction field or a sample solution is tried to be detected is to increase the detection sensitivity by amplifying the infinitesimal substance. For example, a technique in which an infinitesimal nucleic acid strand to be detected is amplified by the PCR (polymerase chain reaction) method has been widely performed.

However, amplification of such an infinitesimal substance requires careful work and also takes a lot of efforts, and further it often needs a special expensive device. Moreover, even if the work is done with caution, a substance other than the target substance may be secondarily generated in the amplification process. At this time, there was a technical problem that detection noise was caused. In addition, there is also a technical problem that it is more difficult to amplify a biological substance such as a protein or a fat than a nucleic acid strand.
US 2004/0101893 A1 provides a method for detection and/or genotyping of nucleic acids that utilizes the specificity of an AP endonuclease.

In light of this, a major object of the invention is to provide a technique capable of detecting an infinitesimal substance with a high sensitivity without amplifying the infinitesimal substance.

### Disclosure of the invention

The present invention first provides a nucleic acid strand for detection having at least a fluorescent substance which can serve as a donor of resonance excitation energy (fluorescence resonance energy), a quencher substance which is located at a position enabling it to receive the resonance excitation energy, and a nucleic acid strand region which is located between the quencher substance and the fluorescent substance and has an enzyme cleavage site, which is an abasic site (AP site), to be cleaved by a double strand-specific AP-endonuclease formed therein, wherein in the nucleic acid strand region a responsive element region which binds to a transcription factor present and wherein the nucleic acid strand region is an oligonucleotide strand.

The "quencher substance" has a broad meaning of a substance having a function of reducing or quenching fluorescence and is not limited narrowly. The "nucleic acid strand" means a polymer (nucleotide strand) of a phosphate of a nucleoside in which a purine or pyrimidine base is linked to a sugar through a glycosidic linkage and widely encompasses oligonucleotides including probe DNA, polynucleotides, DNA in which a purine nucleotide and a pyrimidine nucleotide are polymerized (the full-length or a fragment thereof), cDNA obtained by reverse transcription (cDNA probe), RNA, and the like.

The "endonuclease" is a collective term of nucleases capable of cleaving a nucleic acid at an interior bond. In the invention, this enzyme is used alone or in combination with another enzyme which exhibits a cooperative action. The "nucleic acid strand for detection" is a nucleic acid strand to be used for the purpose of detecting a given substance, a reaction (including a chemical bonding, an interaction, etc.), a structure, or the like. It coincides with such a concept as being called a probe nucleic acid strand in some cases.

As the enzyme cleavage site present in the nucleic acid strand region which constitutes the nucleic acid strand for detection according to the invention an abasic site (AP site) which can be cleaved by a double strand-specific AP-endonuclease is used.

The "AP site (apurinic/apyrimidinic site) " means a site where a base is deleted (lost) in a nucleic acid strand, and the "AP-endonuclease (apurinic/apyrimidinic endonuclease)" is an enzyme having an AP lyase activity to introduce a nick into a nucleic acid strand at an AP site. Some examples thereof are mentioned as follows: endonuclease VI, endonuclease IV (cleavage on the 5' side of the AP site), endonuclease III (cleavage on the 3' side of the AP site), endonuclease S1, endonuclease VIII, formamidopyrimidine-DNA glycosylase (Fpg), OGG1 and the like. In the invention, a particularly useful AP-endonuclease is an enzyme which specifically recognizes a double strand and cleaves one nucleic acid strand having an abasic site at the abasic site.

The number of bases (number of nucleic acid molecules) of the nucleic acid strand according to the invention is not particularly limited, however, the nucleic acid strand is an oligonucleotide strand. Also, the use of the nucleic acid strand is not particularly limited, however, to cite an example, the nucleic acid strand can be used as a probe for detecting a target substance (a substance to be detected). Incidentally, the oligonucleotide strand means a nucleotide polymer consisting of several tens of nucleotides, for example, about 15 to 30 nucleotides. In the nucleic acid strand region, a responsive element region which binds to a given protein is present, and the protein is a transcription factors.

Subsequently, the invention provides a method using a nucleic acid strand comprising at least the following steps (1) and (2): (1) a step of allowing a complementary strand formation reaction involved in the nucleic acid strand for detection as described above; and (2) a step of cleaving a probe nucleic acid strand at the AP-site by allowing the double strand specific AP-endonuclease specific for a double strand obtained by the complementary strand formation reaction to act thereon to effect fragmentation and also dissociating the cleaved strand into single strands thereby amplifying the fluorescence of the fluorescent substance.

The "complementary strand formation reaction" is so-called hybridization between nucleic acids (nucleotide strands), and widely includes complementary binding between DNA-DNA, DNA-RNA, RNA-RNA, and the like.

In the method according to the invention, when it is assumed that the nucleic acid strand for detection in a state where fluorescence is reduced or quenched due to the action of the quencher substance forms a double strand with a nucleic acid strand complementary thereto, the nucleic acid strand for detection is cleaved at the endonuclease cleavage site by the action of the double strand-specific endonuclease and also dissociated into single strands.

In this step of dissociation into single strands, the nucleic acid strand for detection is fragmented into a nucleic acid strand on the fluorescent substance side and a nucleic acid strand on the quencher substance side and then released as single strands. Therefore, the fluorescent substance exists apart from the quencher substance, which results in amplifying the fluorescence (because the effect of the quencher substance is lost).

This method can be performed, for example, under a condition of a temperature not higher than an upper limit temperature at which the complementary strand formation reaction of the nucleic acid strand for detection before the cleavage by the endonuclease can be allowed to proceed and not lower than a temperature at which the complementary strand formation reaction of the fragments of the nucleic acid strand for detection after the cleavage by the endonuclease cannot be maintained or allowed to proceed.

In the invention, by measuring the presence or absence of fluorescence amplification or the fluorescence amplification level (degree of fluorescence amplification), the formation of a double strand (complementary strand) from the nucleic acid strand for detection, i.e., the presence of a target nucleic acid strand complementary to the probe nucleic acid strand, and moreover, the presence of a biological substance other than nucleic acids, the occurrence of a chemical change or a structural change in a biological substance, the presence of a drug candidate substance, and the like can be found.

By using the nucleic acid strand in the invention, an infinitesimal substance can be detected with a high sensitivity without amplifying the infinitesimal substance.

### Brief Description of the Drawings

[Fig. 1] Fig. 1 is a view for illustrating a concept of a nucleic acid strand for detection.
[Fig. 2] Fig. 2 is a view showing an nucleic acid strand for detection in which both fluorescent substance (F) and quencher substance (Q) are bound to sites in the interior of the nucleic acid strand.
[Fig. 3] Fig. 3 is a view showing another nucleic acid strand for detection in which a fluorescent substance (F) is bound to an end site of the nucleic acid strand and the other substance, a quencher substance (Q) is bound to a site in the interior of the nucleic acid strand.
[Fig. 4] Fig. 4 is a view showing still another nucleic acid strand for detection in which a fluorescent substance (F) is bound to a site in the interior of the nucleic acid strand and the other substance, a quencher substance (Q) is bound to an end site of the nucleic acid strand.
[Fig. 5] Fig. 5 is a view showing a function of a nucleic acid strand for detection (P) and a typical application example thereof.
[Fig. 6] Fig. 6 is a view schematically showing a manner in which a nick is formed at an AP site X by an AP-endonuclease E.
[Fig. 7] Fig. 7 is a view schematically showing a state in which fragments (P₁ and P₂) of a nucleic acid strand for detection obtained by cleavage with an AP-endonuclease (E) are dissociated from a nucleic acid strand (T) and released in a reaction field (R) as single strands under an appropriate temperature condition (t).
[Fig. 8] Fig. 8 is a view showing a basic flow of a "cycle reaction" caused by a nucleic acid strand for detection P according to the invention.
[Fig. 9] Fig. 9 is a reference view showing a manner of double strand formation or dissociation of a nucleic acid strand for detection (P) (uncleaved) and fragments (P₁ and P₂) of a nucleic acid strand for detection (obtained by cleavage with an AP-endonuclease E) depending on a change in the temperature condition of a reaction field (R).
[Fig. 10] Fig. 10 is a schematic view showing a concept of a first application example of a method using a nucleic acid strand for detection (P).
[Fig. 11] Fig. 11 is a schematic view showing a concept of a second application example of a method using a nucleic acid strand for detection (P).
[Fig. 12] Fig. 12 is a schematic view showing a concept of a third application example of a method using a nucleic acid strand for detection (P).
[Fig. 13] Fig. 13 is a schematic view showing a concept of a fourth application example of a method using a nucleic acid strand for detection (P).
[Fig. 14] Fig. 14 is a schematic view showing a concept of a fifth application example of a method using a nucleic acid strand for detection (P) according to the invention.
[Fig. 15] Fig. 15 is a schematic view showing a concept of a sixth application example of a method using a nucleic acid strand for detection (P).
[Fig. 16] Fig. 16 is a schematic view showing a concept of a seventh application example of a method using a nucleic acid strand for detection (P) in the case where a substance (for example, a protein Dx) before a structural change is detected.
[Fig. 17] Fig. 17 is a schematic view showing a concept of the seventh application example of the method in the case where a substance (for example, a protein Dy) after a structural change is detected.
[Fig. 18] Fig. 18 is a schematic view showing a concept of an eighth application example of the method in the case where a nucleic acid strand for detection P is used as a probe for a DNA chip.
[Fig. 19] Fig. 19 is a view (a photograph showing results of agarose electrophoresis) showing results obtained by reacting 100 fmol of a target nucleic acid strand and 8 pmol of a nucleic acid strand for detection (having an AP site) with an AP-endonuclease and detecting the cleavage of the nucleic acid strand for detection in Example 1.
[Fig. 20] Fig. 20 is a view (longitudinal axis: fluorescence intensity, horizontal axis: time (min)) showing results obtained by detecting amplification of fluorescence signal over time in Example 2.
[Fig. 21] Fig. 21 is a view (graph) obtained by plotting concentrations of respective target nucleic acid strands and initial reaction velocities using the "Lineweaver Burk Plot" in Example 2.
[Fig. 22] Fig. 22 is a view (graph substituted for drawing) showing results of measurement of an increase in fluorescence of a fluorescent dye (FITC) over time and obtained by plotting increasing ratios of fluorescence per unit time against each time point in Example 3.
[Fig. 23] Fig. 23 is a view (graph substituted for drawing) showing results obtained by detecting a BMAL1-CLOCK complex by changing the number of Hela cells (horizontal axis) in Example 3.
[Fig. 24] Fig. 24 is a view (graph substituted for drawing) showing results obtained by stimulating Hela cells in a culture medium (DMEM culture medium) containing 50 horse serum for 2 hours, recovering the cells at 15 hours, 17.5 hours, 20 hours, and 22.5 hours after initiation of the stimulation, and detecting a BMAL1-CLOCK protein complex in Example 4.
[Fig. 25] Fig. 25 is a view (graph substituted for drawing) showing results obtained by quantitatively determining a Per1 mRNA level in Example 4.
[Fig. 26] Fig. 26 is a view (graph substituted for drawing) showing results obtained by quantitatively determining a Per2 mRNA level in Example 4.

### Best Mode for Carrying Out the Invention

Hereinafter, embodiments of the invention will be described with reference to the attached drawings. However, the concept of the invention is not construed narrowly based on the embodiments described below.

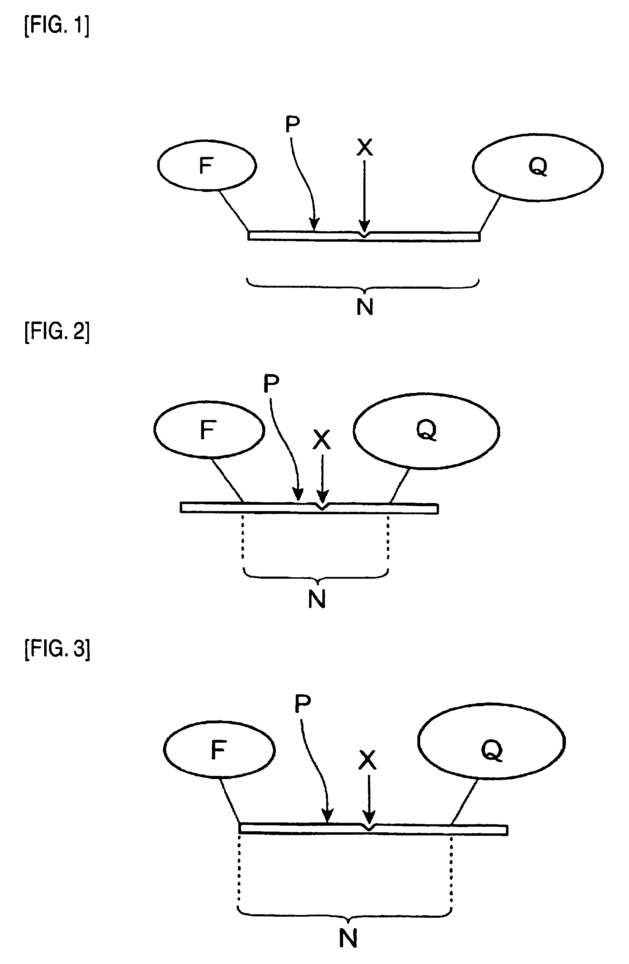

First, Figs. 1 to 4 are views for illustrating a concept of a nucleic acid strand for detection.

A symbol P shown in Figs. 1 to 4 indicates an embodiment of the nucleic acid strand for detection. This nucleic acid strand for detection P has at least a fluorescent substance F which can serve as a donor of resonance excitation energy (fluorescence resonance energy), a quencher substance Q which is located at a position enabling it to receive the resonance excitation energy, a nucleic acid strand region N which is located between the quencher substance and the fluorescent substance, and an enzyme cleavage site (for example, an AP site: in the drawings, it is denoted by a symbol X) present in the nucleic acid strand region N.

For example, in the case where a fluorescent substance F is bound (for labeling) to the 3' end or 5' end of a nucleic acid strand with a given molecular length, and a quencher substance Q is bound (for labeling) to an end opposite to the end to which the fluorescent substance F is bound, the entire region of the nucleic acid strand is located between the fluorescent substance F and the quencher substance Q, therefore, the entire region of the nucleic acid strand corresponds to the nucleic acid region N according to the invention (see Fig. 1).

Further, in a structure in which both or either of the fluorescent substance F and the quencher substance Q are/is bound to a position other than the ends of the nucleic acid strand with a given molecular length, a nucleic acid strand region located between both substances F and Q corresponds to the nucleic acid region N (see Figs. 2 to 4).

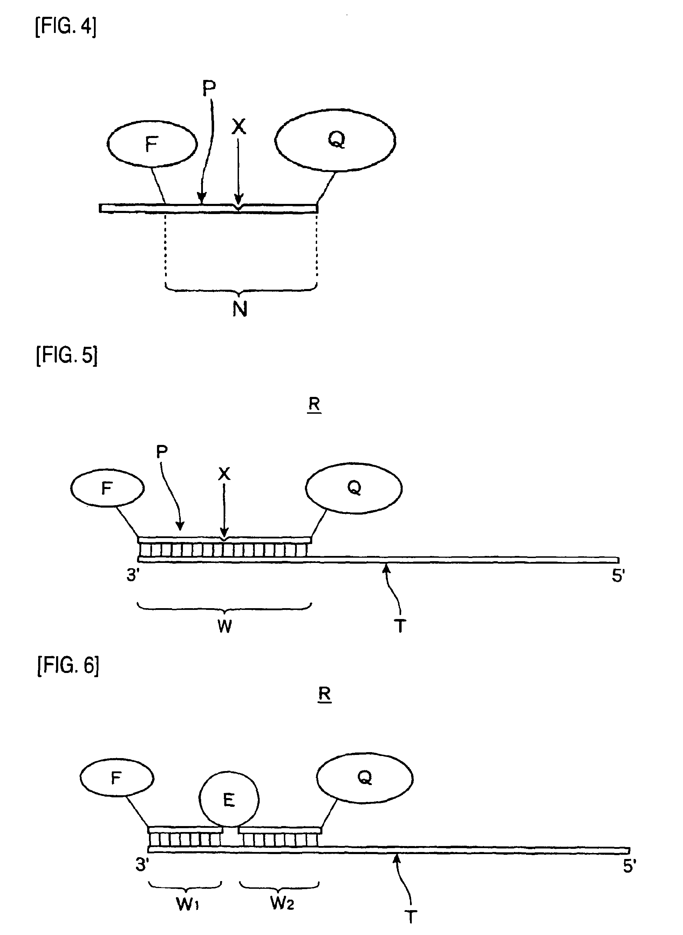

Specifically, Fig. 2 shows a possibility in which both fluorescent substance F and quencher substance Q are bound to sites in the interior of the nucleic acid strand; Fig. 3 shows another possibility in which a fluorescent substance F is bound to an end site of the nucleic acid strand and the other substance, a quencher substance Q is bound to a site in the interior of the nucleic acid strand; and Fig. 4 shows still another possibility in which a fluorescent substance F is bound to a site in the interior of the nucleic acid strand and the other substance, a quencher substance Q is bound to an end site of the nucleic acid strand.

Fig. 5 is a view showing a function of the nucleic acid strand for detection P and a typical application example thereof. In the example shown in Fig. 5, an example in which by using the nucleic acid strand for detection P, the presence of a nucleic acid strand T having a nucleotide sequence complementary to that of the nucleic acid strand region N or abundance thereof is detected is shown. In this example, the nucleic acid strand for detection P shown in Fig. 1 is illustrated as the typical example (the nucleic acid strand for detection P shown in any of Figs. 2 to 4 can also be employed).

In the nucleic acid strand for detection P, fluorescence to be emitted from the fluorescent substance F is reduced or quenched by the action of the quencher substance Q before it is subjected to a given enzymatic treatment (which will be mentioned below). When a sample solution is introduced into a reaction field R in which the nucleic acid strand for detection P in this state is present, if the nucleic acid strand T having a nucleotide sequence complementary to that of the nucleic acid strand region N of the nucleic acid strand for detection P is present in the sample solution, the nucleic acid strand region N and the nucleic acid strand T form a double strand (complementary strand) (complementary strand formation step). Incidentally, a symbol W in Fig. 5 denotes a region of the double strand (complementary strand) formed through the complementary strand formation step (hereinafter, the same shall apply).

When a double strand-specific AP-endonuclease E is introduced into the reaction field R after or concurrently with the complementary strand formation step (see Fig. 6), the AP-endonuclease E recognizes an AP site X present in the nucleic acid strand region N (of the nucleic acid strand for detection P) which forms the complementary strand W and introduces a nick into the AP site X and cleaves the nucleic acid strand for detection P into a fragment at the fluorescent substance F side and a fragment at the quencher substance Q side. That is, the double strand (complementary strand) region W is cleaved into short strands, a double strand W₁ at the fluorescent substance F side and a double strand W₂ at the quencher substance Q side. Incidentally, Fig. 6 is a view schematically showing a manner in which a nick is formed at the AP site X by the AP-endonuclease E.

Here, the double strand (complementary strand) region W (see Fig. 5) and the double strands W₁ and W₂ (see Fig. 6) obtained by cleavage into short strands with the AP-endonuclease E have different strand lengths, therefore, they have different tₘ (melting temperature). That is, relationships of the respective tₘ temperatures are as follows: W > W₁, and W > W₂.

Accordingly, a condition of a buffer solution temperature in the reaction field R is set to a condition of an appropriate temperature (t) which is not higher than a temperature tₐ at which the complementary strand formation reaction of the nucleic acid strand for detection P before the cleavage by the AP-endonuclease E can be allowed to proceed or maintained and is not lower than a temperature t_{b} at which the complementary strand formation reaction of the fragments P₁ and P₂ (see Fig. 6) of the nucleic acid strand for detection after the cleavage by the AP-endonuclease E cannot be maintained or allowed to proceed, that is, t is set as follows: t_{b} ≤ t ≤ tₐ.

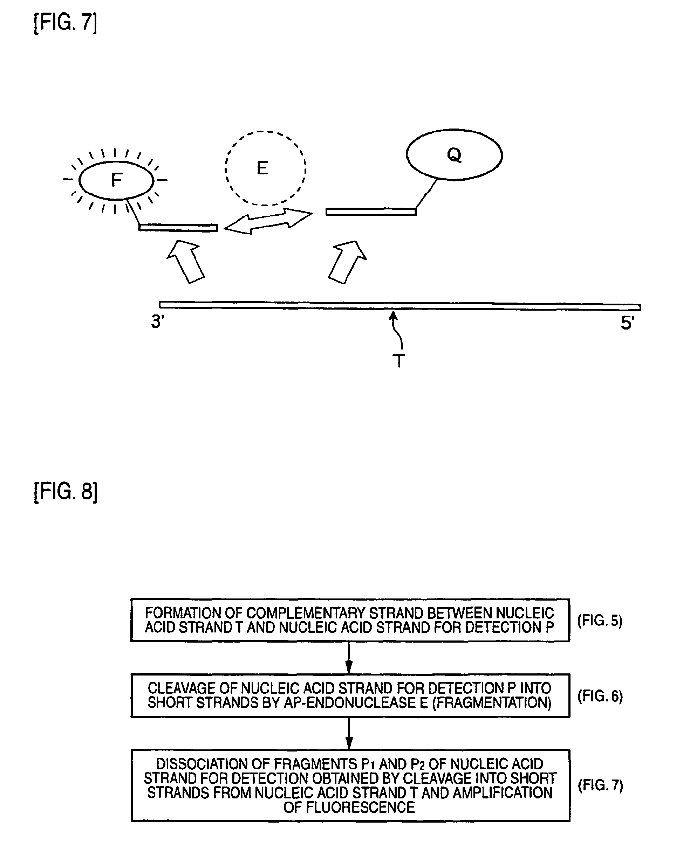

Fig. 7 schematically shows a state in which the fragments P₁ and P₂ (see Fig. 6) of the nucleic acid strand for detection obtained by cleavage with the AP-endonuclease E are dissociated from the nucleic acid strand T and released in the reaction field R as single strands under the appropriate temperature condition t.

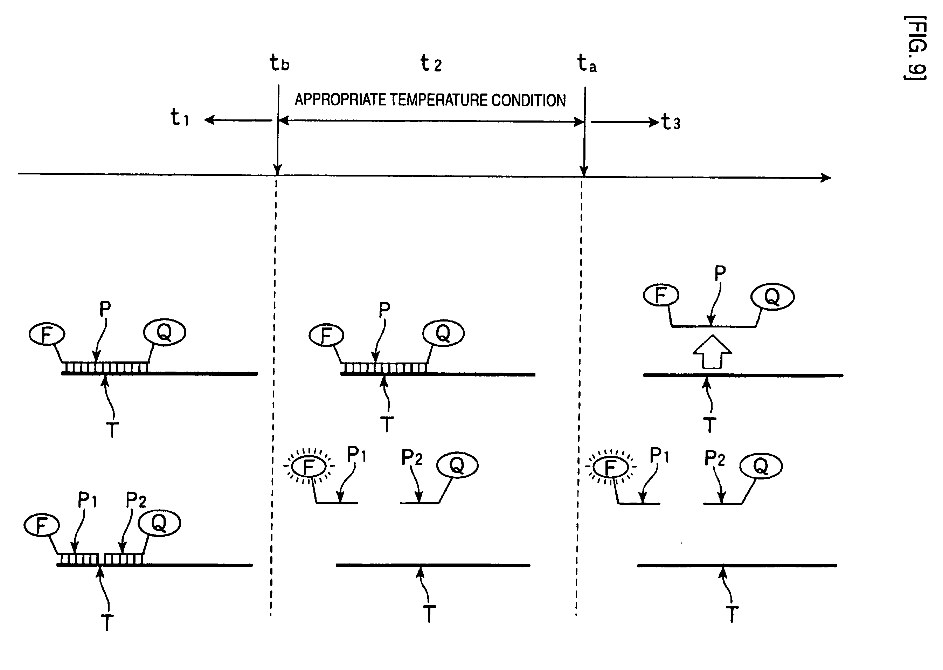

Further, Fig. 9 is a reference view showing a manner of double strand formation or dissociation of the nucleic acid strand for detection P (uncleaved) and the fragments P₁ and P₂ of the nucleic acid strand for detection (obtained by cleavage with the AP-endonuclease E) depending on a change in the temperature condition of the reaction field R.

To be more specific with respect to Fig. 9, under a condition of a temperature t₁ lower than the temperature t_{b}, double strand formation of all of the nucleic acid strand for detection P and the fragments P₁ and P₂ of the nucleic acid strand for detection can be allowed to proceed and also the formed double strand can be maintained.

Under a condition of an appropriate temperature t₂ (t_{b} ≤ t₂ ≤ tₐ), the complementary strand formation reaction of the nucleic acid strand for detection P can be allowed to proceed and the formed complementary strand can be maintained. On the other hand, the fragments P₁ and P₂ of the nucleic acid strand for detection obtained by cleavage into short strands with the AP-endonuclease E are dissociated from the nucleic acid strand T into single strands, respectively. By this dissociation into single strands, the fluorescent substance F exists apart from the quencher substance Q, which results in amplifying the fluorescence (because the effect of the quencher substance Q is lost) (see Fig. 9).

Further, under a condition of a temperature t₃, also the nucleic acid strand for detection P is dissociated into a single strand, and the fragments P₁ and P₂ of the nucleic acid strand for detection obtained by cleavage into short strands with the AP-endonuclease E are also dissociated from the nucleic acid strand T into single strands, respectively (see Fig. 9).

Here, the invention has an advantage that by introducing the nucleic acid strand for detection P in an excess amount relative to the nucleic acid strand T into the reaction field R, an amplified strong fluorescence signal can be obtained even from the infinitesimal nucleic acid strand T present in the reaction field R by repeating a series of reactions including "formation of a complementary strand between the nucleic acid strand T and the nucleic acid strand for detection P (see Fig. 5)" → "cleavage of the nucleic acid strand for detection P into short strands by the AP-endonuclease E (see Fig. 6)" → "dissociation of the fragments P₁ and P₂ of the nucleic acid strand for detection obtained by cleavage into short strands from the nucleic acid strand T and amplification of fluorescence accompanying the dissociation (see Fig. 7)" a number of times by the nucleic acid strand for detection P present in the reaction field R (hereinafter, conveniently referred to as "cycle reaction"). Incidentally, Fig. 8 is a view showing a basic flow of this cycle reaction.

That is, a strong fluorescence signal derived from the fluorescent substance F (which ceases to be quenched by the quencher substance Q) showing the presence of the nucleic acid strand T can be obtained from the reaction field R in an infinitesimal amount of the nucleic acid strand T as it is without taking the trouble to amplify the amount of the nucleic acid strand T present in the reaction field R, and thus, the nucleic acid strand T can be detected with a high sensitivity.

Accordingly, in the invention, it is important that the temperature condition of the reaction field R is set so as to enable the complementary strand formation between the nucleic acid strand T and the nucleic acid strand for detection P to proceed, therefore, the condition of the temperature t₃ exceeding tₐ as shown in Fig. 9 is not preferred.

Consequently, as described above, the preferred temperature condition in the invention is a temperature not higher than the temperature tₐ at which the complementary strand formation reaction of the nucleic acid strand for detection P before the cleavage by the AP-endonuclease E can be allowed to proceed or maintained and is not lower than the temperature t_{b} at which the complementary strand formation reaction of the fragments P₁ and P₂ (see Fig. 6) of the nucleic acid strand for detection after the cleavage by the AP-endonuclease E cannot be maintained or allowed to proceed (that is, t_{b} ≤ t ≤ tₐ) (see Fig. 9 again).

Incidentally, a case in which the tₘ temperatures for the fragments P₁ and P₂ are different from each other because the strand lengths of the fragments P₁ and P₂ (see Fig. 6) of the nucleic acid strand for detection are different from each other is assumed, and in this case, by adopting the higher tₘ as the lower limit temperature t_{b} of the set temperature condition, both fragments P₁ and P₂ of the nucleic acid strand for detection can be dissociated from the nucleic acid strand T. In this manner, the above-mentioned repetitive reaction can be performed.

Hereinafter, several application examples of the method using the nucleic acid strand for detection P will be described. The application examples of the method described hereunder are only for illustrative purposes, and the invention is not narrowly limited to these.

A feature common to all application examples of the method illustrated hereunder resides in the point that an infinitesimal substance, an infinitesimal reaction or interaction, or a structural change in a substance can be detected with a high sensitivity through the above-mentioned "cycle reaction" involved in an excess amount of the nucleic acid strand for detection P introduced into the reaction field. A detailed description on a case-by-case basis of this point is omitted to avoid duplication.

### <First application example of method as background art for understand by the invention>

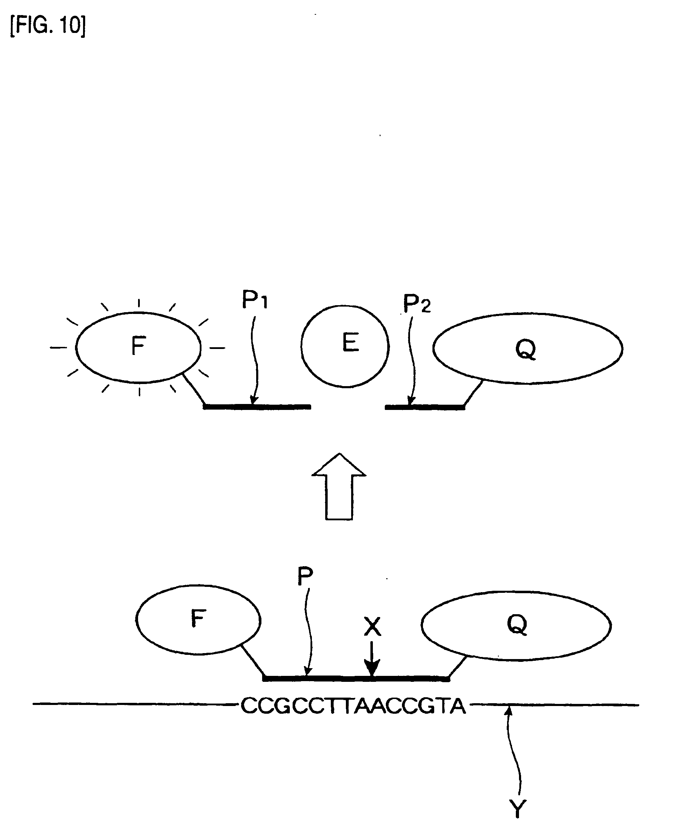

Fig. 10 is a schematic view showing a concept of a first application example of the method using a nucleic acid strand for detection P. This first application example of the method is useful for a case in which whether or not a target nucleotide sequence region is present in a nucleotide sequence of a nucleic acid strand Y is verified and the like. To cite an example, whether or not a responsive element region specific for a given transcription factor is present in a promoter region located upstream of a gene can be confirmed.

### <Second application example of method as background art for undertandly the invention>

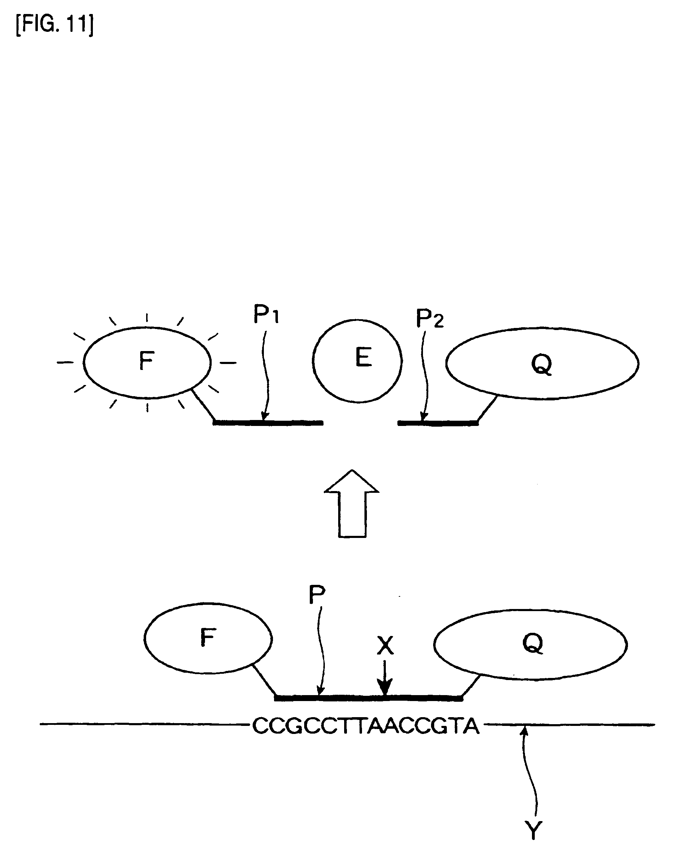

Fig. 11 is a schematic view showing a concept of a second application example of the method using a nucleic acid strand for detection P. According to this second application example of the method, whether or not a substance (such as a protein) to be detected is present on a surface S of a solid phase (such as a substrate, a bead, or a support) can be detected.

More specifically, a sample solution is introduced into a reaction field R which a surface S faces, and subsequently, a nucleic acid strand Z₁ having a nucleotide sequence specifically binding to a substance M to be detected is introduced, and thereafter or concurrently therewith, a nucleic acid strand for detection P having a nucleic acid strand region N complementary to the above nucleotide sequence is introduced, and then, the reaction field R is washed with a solution. Then, further the nucleic acid strand for detection P and a double strand-specific AP-endonuclease E are introduced into the reaction field R.

If the substance M is present in the sample solution, the nucleic acid strand Z₁ and the nucleic acid strand for detection P which have formed a complementary strand are bound to the substance M. Therefore, the AP-endonuclease E acts on the complementary strand and introduces a nick into (the AP site X in the nucleic acid strand region N which constitutes) the complementary strand and dissociates it into single strands. Accordingly, even if the content of the substance M is infinitesimal, a strong fluorescence signal from the fluorescent substance F which constituted the nucleic acid strand for detection P can be obtained. In this manner, the substance M can be detected with a high sensitivity.

### <Third application example of method as background art for undertanding the invention>

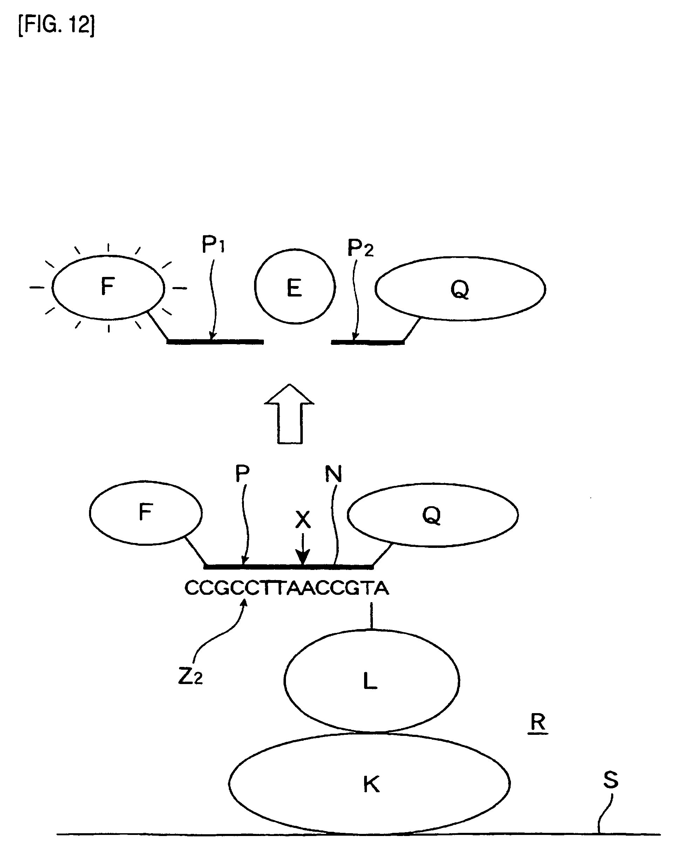

Fig. 12 is a schematic view showing a concept of a third application example of the method using a nucleic acid strand for detection P. According to this third application example of the method, a reaction or an interaction of a substance L with a substance K immobilized on a surface S of a solid phase (such as a substrate or a bead) can be detected.

More specifically, a substance K has been immobilized on a surface S in advance, and a sample solution containing a substance L for which the presence or absence of a reaction or an interaction with the substance K is verified is introduced into a reaction field R which the surface S faces, and then, the reaction field R is washed with a solution. The substance L has been labeled with a nucleic acid strand Z₂ having a given nucleotide sequence in advance.

Subsequent to washing with a solution, a nucleic acid strand for detection P having a nucleic acid strand region N complementary to the nucleotide sequence of the nucleic acid strand Z₂ is introduced, and then, a double strand-specific AP-endonuclease E is introduced into the reaction field R.

If the substance K and the substance L are reacted and bound to or interact with each other, the nucleic acid strand Z₂ with which the substance L has been labeled and (the nucleic acid strand region N of) the nucleic acid strand for detection P form a complementary strand in the reaction field R. Therefore, the double strand-specific AP-endonuclease E acts on the complementary strand and introduces a nick into (the AP site X in the nucleic acid strand region N which constitutes) the complementary strand and dissociates it into single strands. Accordingly, even if the reaction or interaction level between the substance K and the substance L is infinitesimal, a strong fluorescence signal from the fluorescent substance F which constituted the nucleic acid strand for detection P can be obtained.

In this manner, a reaction (such as an antigen-antibody reaction) or an interaction (such as a protein-protein interaction or a reaction between a lipid and a binding molecule) between the substance K and the substance L can be detected with a high sensitivity.

### <Fourth application example of method as background art for undertanding the invention>

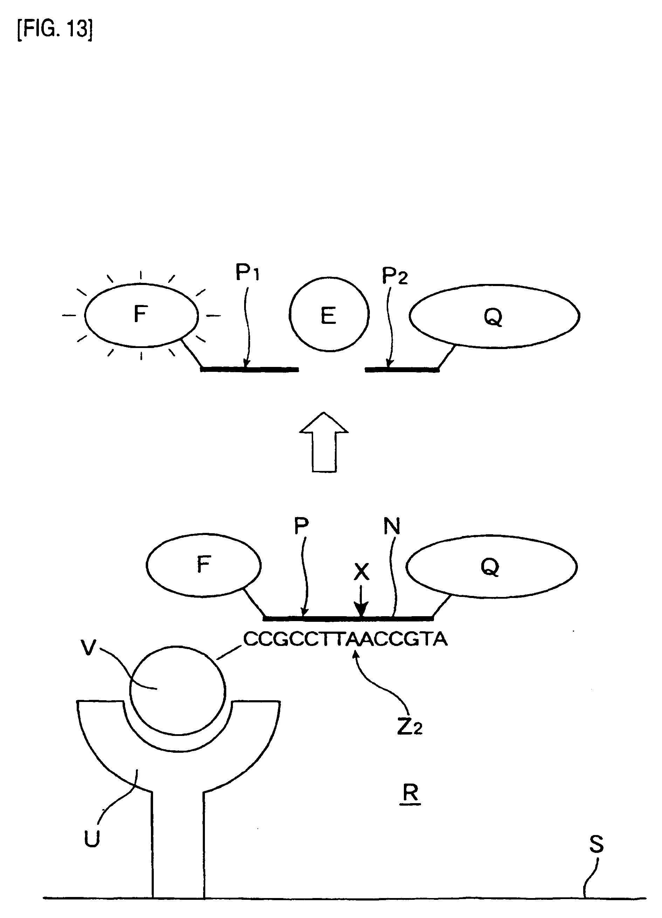

Fig. 13 is a schematic view showing a concept of a fourth application example of the method using a nucleic acid strand for detection P. According to this fourth application example of the method, a low-molecular compound which can be a drug candidate, for example, a compound which can be an agonist or an antagonist by binding to a receptor can be detected or screened with a high sensitivity.

More specifically, for a substance U present on a surface S of a solid phase such as a substrate, a sample solution containing a candidate substance V which can be bound to or interact with the substance U is introduced, and then, a reaction field R is washed with a solution. The substance V has been labeled with a nucleic acid strand Z₂ having a specific nucleotide sequence in advance.

Subsequent to washing with a solution, a nucleic acid strand for detection P having a nucleic acid strand region N complementary to the nucleotide sequence of the nucleic acid strand Z₂ is introduced, and then, a double strand-specific AP-endonuclease E is introduced into the reaction field R.

If the substance U and the candidate substance V are bound to each other, the nucleic acid strand Z₂ with which the candidate substance V has been labeled and (the nucleic acid strand region N of) the nucleic acid strand for detection P form a complementary strand in the reaction field R. Therefore, the double strand-specific AP-endonuclease E acts on the complementary strand and introduces a nick into (the AP site X in the nucleic acid strand region N which constitutes) the complementary strand and dissociates it into single strands. Accordingly, even if the amount of binding between the substance U and the candidate substance V is infinitesimal, a strong fluorescence signal from the fluorescent substance F which constituted the nucleic acid strand for detection P can be obtained.

In this manner, the binding between the substance U and the candidate substance V can be detected with a high sensitivity. By using this detection technique, a drug candidate substance can be screened.

### <Fifth application example of method>

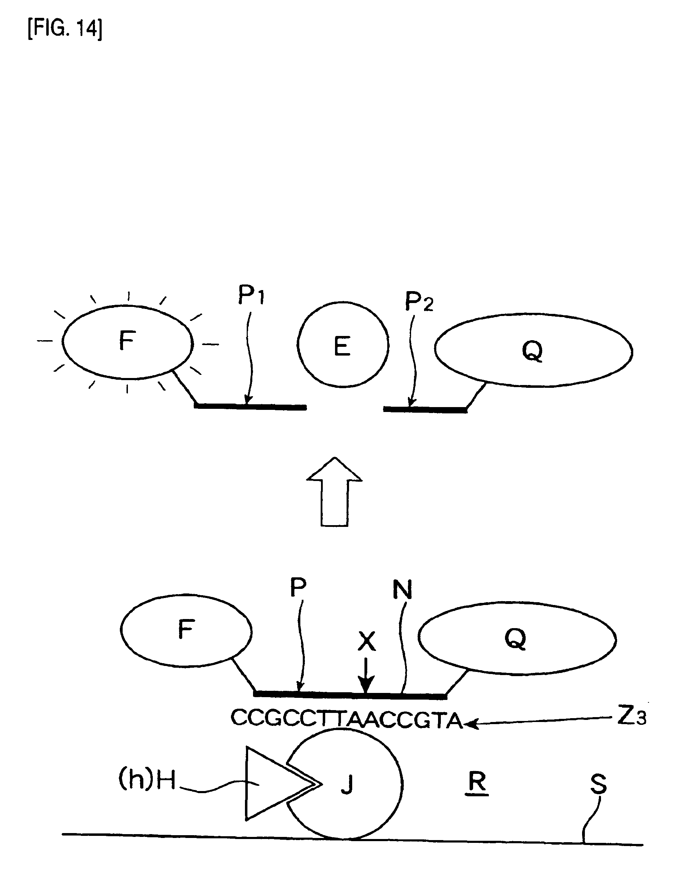

Fig. 14 is a schematic view showing a concept of a fifth application example of the method using a nucleic acid strand for detection P according to the invention. According to this fifth application example of the method, for example, an environmental hormone (endocrine disruptor) can be detected or screened.

First, a certain type of transcription factor J (a DNA-binding protein) has a function as follows. The transcription factor J changes in its structure when a hormone H binds thereto, whereby it binds to a responsive element region located upstream of a gene and promotes the transcription of the gene. In general, an endocrine disruptor which is called an environmental hormone is a substance that behaves like the hormone H in the body.

In the invention, a transcription factor (nuclear receptor) J has been allowed to exist (for example, immobilized) on a surface S of a solid phase such as a substrate or a bead in advance, and also at least a nucleic acid strand Z₃ corresponding to the responsive element region and a nucleic acid strand for detection P having a nucleic acid strand region N complementary to the nucleotide sequence of the nucleic acid strand Z₃ have been allowed to exist in advance. Into a reaction field R containing such substances, a hormone-like substance h to be verified as to whether or not it acts as an endocrine disruptor is introduced.

To be more specific, if the hormone-like substance h actually has a function similar to that of the hormone H, the structure of the transcription factor J is changed by the action of the hormone-like substance h. Into the reaction field R in which the transcription factor J undergoing this structural change is present, the nucleic acid strand Z₃ corresponding to the responsive element region and the nucleic acid strand for detection P having the nucleic acid strand region N which forms a double strand with the nucleic acid strand Z₃ are introduced simultaneously. Subsequently, the reaction field R is washed with a solution, whereby excess nucleic acid strand Z₃ present in the reaction field R in a free form is removed from the reaction field R. Then, further the nucleic acid strand for detection P and also a double strand-specific AP-endonuclease E are introduced into the reaction field R.

If the nucleic acid strand for detection P binding to the transcription factor J via the nucleic acid strand Z₃ are present in the reaction field R, the double strand-specific AP-endonuclease E acts on a complementary strand of the nucleic acid strand Z₃ and (the nucleic acid strand region N of) the nucleic acid strand for detection P and introduces a nick into (the AP site X in the nucleic acid strand region N which constitutes) the complementary strand and dissociates it into single strands.

Accordingly, even if the amount of the hormone-like substance h in the sample solution is infinitesimal, a strong fluorescence signal from the fluorescent substance F which constituted the nucleic acid strand for detection P can be obtained. In this manner, the hormone-like substance h can be detected with a high sensitivity. By using this detection technique, an endocrine disruptor can also be screened.

### <Sixth application example of method as background art for undertanding the invention>

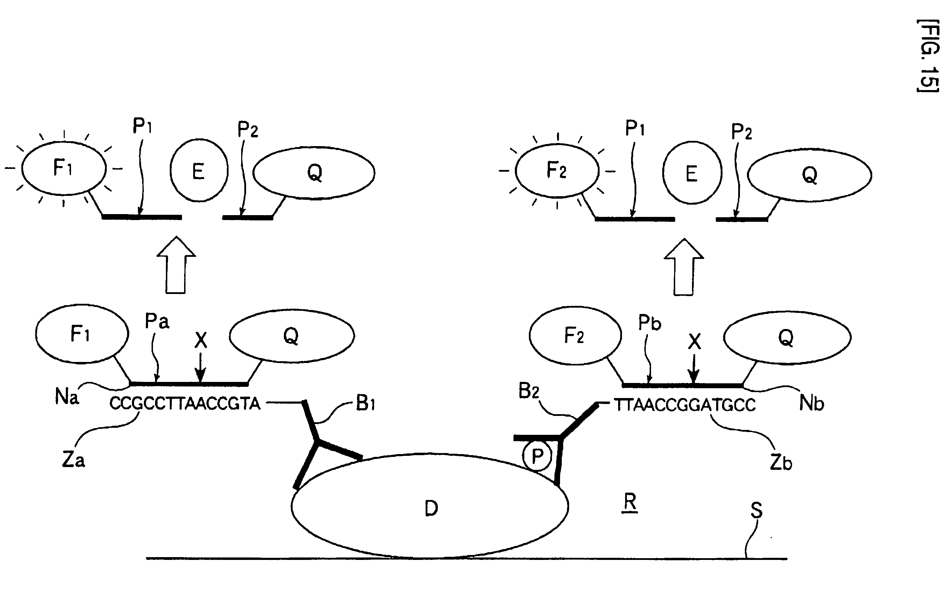

Fig. 15 is a schematic view showing a concept of a sixth application example of the method using a nucleic acid strand for detection P. According to this sixth application example of the method, for example, a modification reaction after translation of a protein can be detected or the like.

First, a protein may sometimes be modified through an enzymatic reaction or the like after translation. For example, phosphorylation is a typical example. In the invention, a protein D to be detected has been allowed to exist (for example, immobilized) on a surface S of a solid phase such as a substrate in advance, and also an antibody B₁ which specifically binds to an unmodified (for example, unphosphorylated) portion of the protein D and an antibody B₂ which specifically binds to a modified (for example, phosphorylated) portion of the protein D have also been allowed to exist in advance. The antibody B₂ and antibody B₂ have been labeled with a nucleic acid strand Za and a nucleic acid strand Zb in advance, respectively, each of which is composed of a specific nucleotide sequence (see Fig. 15).

Into a reaction field R containing such substances, a nucleic acid strand for detection Pa having a nucleic acid strand region Na complementary to the nucleic acid strand Za and a nucleic acid strand for detection Pb having a nucleic acid strand region Nb complementary to the nucleic acid strand Zb are introduced at the same time. Incidentally, the nucleic acid strand for detection Pa has been labeled with a fluorescent substance F₁ capable of emitting fluorescence of a predetermined wavelength and the nucleic acid strand for detection Pb has been labeled with a fluorescent substance F₂ capable of emitting fluorescence of a wavelength different from that of the fluorescent substance F₁.

Subsequent to washing of the reaction field R with a solution, a double strand-specific AP-endonuclease E is introduced into the reaction field R to introduce a nick into both of the nucleic acid strand region Na of the nucleic acid strand for detection Pa which forms a double strand and the nucleic acid strand region Nb of the nucleic acid strand for detection Pb which forms a double strand thereby cleaving them into short strands. The short strands are dissociated into single strands to achieve fluorescence amplification.

In principle, the nucleic acid strand for detection Pa binds to all protein D molecules present in the reaction field R via the strand complementary thereto. Therefore, by measuring the (amplified) fluorescence signal derived from the fluorescent substance F₁ which constituted the nucleic acid strand for detection Pa, the number of all protein D molecules present in the reaction field R can be predicted.

Further, the nucleic acid strand for detection Pb binds to modified (for example, phosphorylated) protein D via the strand complementary thereto among all protein D molecules present in the reaction field R. Therefore, by measuring the (amplified) fluorescence signal derived from the fluorescent substance F₂ which constituted the nucleic acid strand for detection Pb, the number of modified protein D molecules can be predicted.

According to such a technique, a ratio of modified protein D can be predicted based on the number of all protein D molecules and the number of modified protein D molecules present in the reaction field R.

### <Seventh application example of method as background art for undertanding the invention>

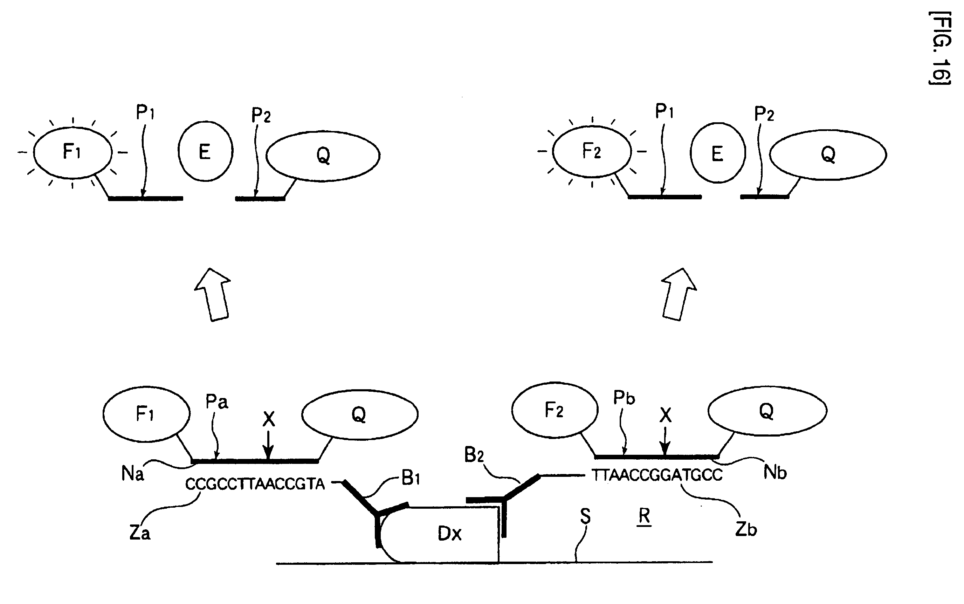

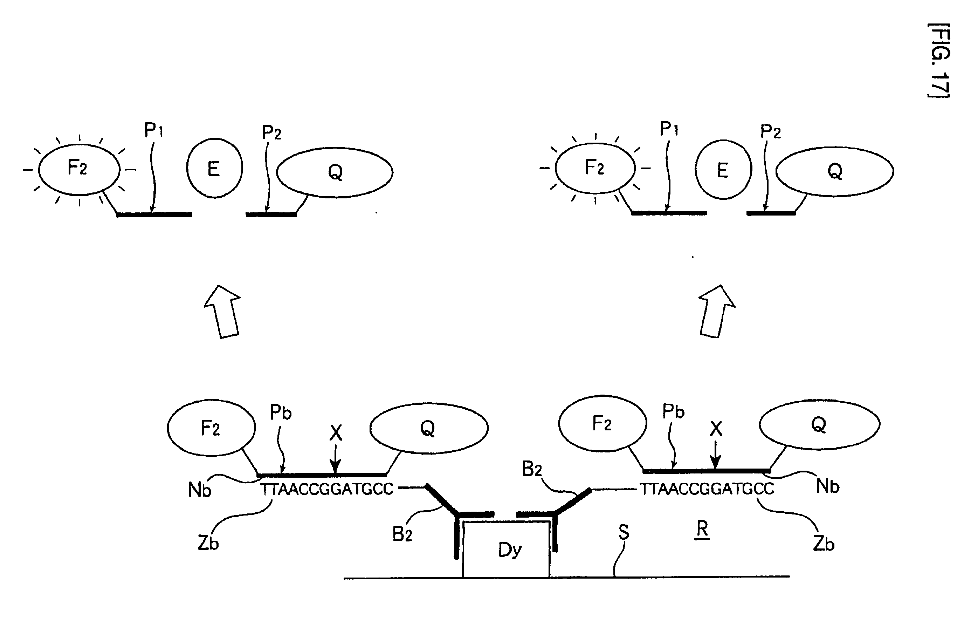

Figs. 16 and 17 are schematic views showing a concept of a seventh application example of the method using a nucleic acid strand for detection P. Fig. 16 is a schematic view in the case where a substance (for example, a protein) before a structural change is detected; and Fig. 17 is a schematic view in the case where a substance (for example, a protein) after a structural change is detected. According to this seventh application example of the method, a structural change in a substance, a numerical ratio of the structural change, or the like can be detected.

For example, a protein may change in its conformation by the action of another factor to exhibit a distinctive function. Accordingly, by detecting such a structural change in a substance, the function or activity of the substance can be known. Hereinafter, a specific description will be made with reference to Figs. 16 and 17.

A symbol Dx shown in Fig. 16 denotes a protein before a structural change, and a symbol Dy shown in Fig. 17 denotes a protein after a structural change. First, by referring to Fig. 16, an antibody B₁ (labeled with a nucleic acid strand Za) which specifically binds to a structural portion d1 of the protein Dx and an antibody B₂ (labeled with a nucleic acid strand Zb) which specifically binds to a structural portion d2 of the protein Dx and two types of nucleic acid strands for detection Pa and Pb with different fluorescence wavelengths are introduced into a reaction field R in which the protein Dx immobilized on a surface S or the like is present.

A nucleic acid strand region Na which constitutes the nucleic acid strand for detection Pa is complementary to the nucleic acid strand Za with which the antibody B₁ has been labeled and therefore forms a double strand. On the other hand, a nucleic acid strand region Nb which constitutes the nucleic acid strand for detection Pb is complementary to the nucleic acid strand Zb with which the antibody B₂ has been labeled and therefore forms a double strand (see Fig. 16).

When a double strand-specific AP-endonuclease E is introduced into such a reaction field R, the nucleic acid strand regions Na and Nb are nicked and dissociated into single strands, respectively. As a result, the fluorescence signals of respective wavelengths from the fluorescent substances F₁ and F₂ are emitted. From this, it is found that a structural change in the protein Dx present on the surface S does not occur.

On the other hand, by referring to Fig. 17, an antibody B₁ (labeled with a nucleic acid strand Za) which specifically binds to a structural portion d1 of the protein Dx (see Fig. 16) and an antibody B₂ (labeled with a nucleic acid strand Zb) which specifically binds to a structural portion d2 (in this case, this portion is the same as the structural portion d2 of the protein Dx) of the protein Dy and two types of nucleic acid strands for detection Pa and Pb with different fluorescence wavelengths are introduced into a reaction field R in which the protein Dy (a protein in which a structural change has occurred) immobilized on a surface S or the like is present.

Between the antibodies B₁ and B₂, only the antibody B₂ binds to the protein Dy in which the structural portion d1 has been lost due to the occurrence of a structural change. Therefore, only the nucleic acid strand region Nb which constitutes the nucleic acid strand for detection Pb complementarily binds to the nucleic acid strand Zb attached to the antibody B₂ for labeling and forms a double strand.

When a double strand-specific AP-endonuclease E is introduced into such a reaction field R, only the nucleic acid strand region Nb is nicked and dissociated into single strands. As a result, only the fluorescence signal from the fluorescent substance F₂ is emitted (see Fig. 17).

From this, it is found that the protein Dy present on the surface S is modified such that a structural change occurs and is distinct from the protein Dx in which a structural change does not occur. According to this method, by analyzing a fluorescence signal, it is also possible to detect both proteins Dx and Dy simultaneously. To cite an example, this method can be applied not only to detection of infinitesimal structural change in β-amyloid, but also to diagnosis of Alzheimer's disease.

### <Eighth application example of method as background art for undertanding the invention>

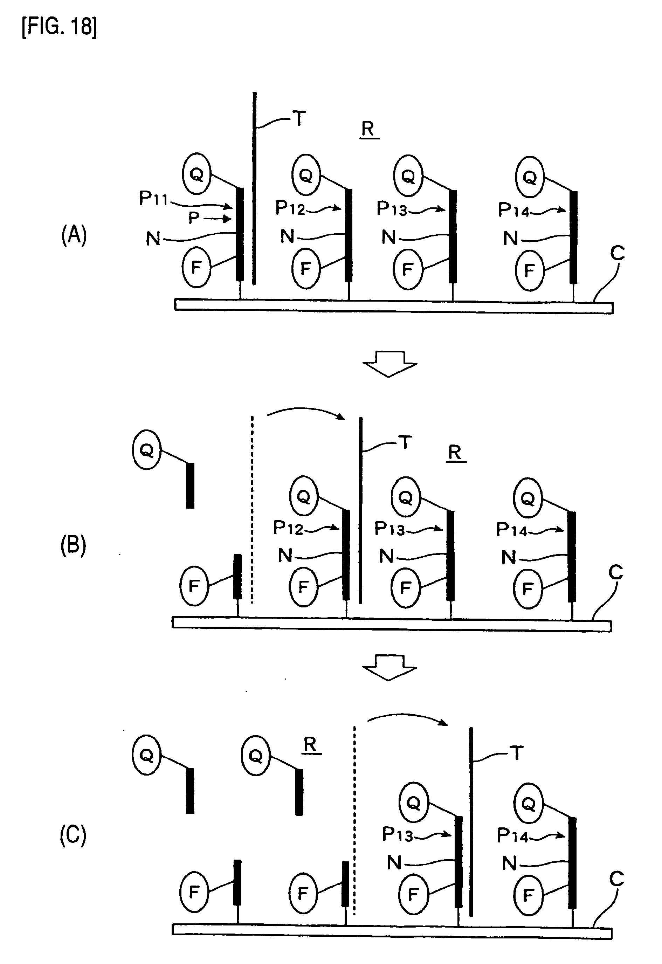

Fig. 18 is a schematic view showing a concept of a seventh application example using a nucleic acid strand for detection P. This seventh application example is an example using the nucleic acid strand for detection P as a probe for a DNA chip. The nucleic acid strand for detection P according to the invention can also be applied to a sensor technique other than the DNA chip.

Fig. 18 shows a state in which nucleic acid strands for detection P (P₁₁, P₁₂, P₁₃, P₁₄) are immobilized on a substrate surface denoted by a symbol C. Figs. 18(A) to 18(C) schematically show the progress of a reaction over time on the substrate surface C.

On the substrate surface C, a series of reactions (a cycle reaction) as follows occurs in chains. When a nucleic acid strand T to be targeted is introduced into a reaction field R, the nucleic acid strand T forms a complementary strand with a nucleic acid strand region N of each of the nucleic acid strands for detection P₁₁, P₁₂, P₁₃ and P₁₄ immobilized on the substrate surface C one after another over time. Subsequently, when the nucleic acid strand region N is nicked (cleaved into short strands) by a double strand-specific AP-endonuclease E (not shown in Fig. 18), fragments of the nucleic acid strands for detection cleaved into short strands are dissociated from the nucleic acid strand T, and accompanied by the dissociation, fluorescence is amplified.

As can be seen from the reaction example shown in Fig. 18, by using the nucleic acid strand for detection P as a probe for a DNA chip, one molecule of the nucleic acid strand T to be targeted is used in a plurality of probes (P₁₁, P₁₂, P₁₃, and P₁₄ in the example shown in Fig. 18). Therefore, it is possible to achieve detection with a high sensitivity. Further, by observing hybridization to a DNA chip and probe cleavage over time based on amplification of fluorescent dye, it is possible to measure the concentration of a target from a reaction velocity.

In a conventional DNA chip, it was necessary to label a target nucleic acid strand T with a fluorescent dye, however, this labor can be omitted in the invention. Further, a conventional DNA chip had a problem that because a hybridized target nucleic acid strand T was in a state of being immobilized on a probe, an apparent concentration of the target nucleic acid strand T around a reaction field of hybridization was decreased, and the efficiency of hybridization was decreased. However, when the nucleic acid strand for detection P is used, the target nucleic acid strand T is not immobilized, therefore, an apparent concentration of the target nucleic acid strand T around a reaction field of hybridization is not decreased, and the efficiency of hybridization can be expected to be improved.

### Examples

In order to confirm a function of a nucleic acid strand for detection according to the invention and usefulness of a detection technique using the nucleic acid strand for detection, the following Experimental examples 1 to 4 were performed.

### (Experimental example 1)

First, it was verified that when an infinitesimal target nucleic acid strand (SEQ ID NO: 1) is present in a reaction field, a cycle reaction (see Fig. 8 again) involved in an excess amount of a nucleic acid strand for detection (a nucleic acid strand region: SEQ ID NO: 2, see Fig. 1 and the like again for its structure) introduced into the reaction field occurs and a large amount of fragments of the nucleic acid strand for detection are generated by an action of an AP-endonuclease (SEQ ID NO: 3). A symbol "n" in the sequence represented by SEQ ID NO: 2 denotes an AP site, a site where a base is deleted.

The AP-endonuclease used in this experiment is a human AP-endonuclease (APE-1), the fluorescent substance used in the nucleic acid strand for detection is a fluorescent dye FAM (bound to the 5' end), and the quencher substance is TAMRA (bound to the 3' end).

The AP-endonuclease was added to a reaction field in which 100 fmol of a target nucleic acid strand and 8 pmol of the nucleic acid strand for detection (having an AP site) were present and reacted with them. In the presence of the AP-endonuclease, the reaction was performed at 37, 42, and 47°C which fell within an appropriate temperature range, and these resulting reaction mixtures together with a reaction mixture without the addition of AP-endonuclease were subjected to agarose electrophoresis and separated according to the size of the nucleic acid strands for detection and detected by the bound fluorescent dye.

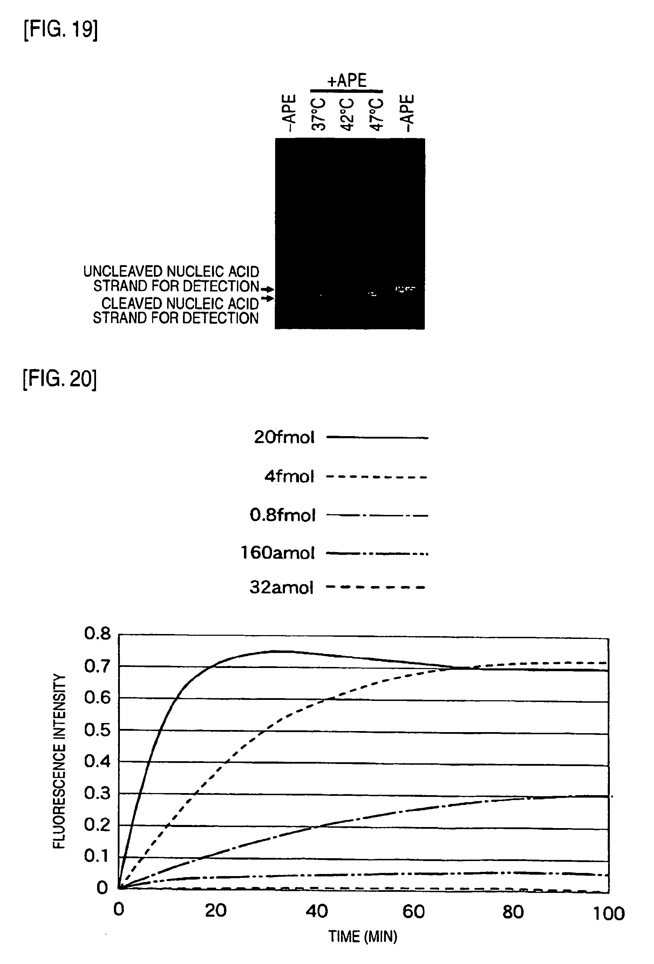

In the separation according to the size of the nucleic acid strands by the agarose electrophoresis, a smaller-sized nucleic acid strand migrates faster (a band migrates downward). In the reaction mixtures with the addition of AP-endonuclease (lanes 2 to 4), the bands migrated downward compared with the reaction mixture without the addition of AP-endonuclease (lanes 1 and 5) (see Fig. 19). From this, it is apparent that the nucleic acid strand for detection used in this experiment was cleaved by the AP-endonuclease.

As is apparent from the results of this Experimental example 1, it was found that the nucleic acid strand for detection in an amount 80 times the amount of the target nucleic acid strand was completely cleaved. That is, it was revealed that a cycle reaction in which a series of reactions comprising "formation of a complementary strand between the target nucleic acid strand and the nucleic acid strand for detection" → "cleavage of the nucleic acid strand for detection into short strands by the AP-endonuclease (fragmentation)" → "dissociation of the fragments of the nucleic acid strand for detection obtained by cleavage into short strands from the target nucleic acid strand" is repeated a number of times by a large amount of the nucleic acid strand for detection present in the reaction field occurs (see Fig. 8 again).

### (Experimental example 2)

In this Experimental example 2, to a different concentration of a target nucleic acid strand (SEQ ID NO: 1), 8 pmol of a nucleic acid strand for detection (having an AP site) (fluorescent substance: FAM (bound to the 5' end), quencher substance: TAMRA (bound to the 3' end), nucleotide sequence of a nucleic acid strand region: SEQ ID NO: 2) was introduced, and further an AP-endonuclease was reacted with the strand, and then, amplification of fluorescence signal was detected over time. The view shown in Fig. 20 is a graph showing the results (longitudinal axis: fluorescence intensity, horizontal axis: time (min)). The concentrations of the target nucleic acid strand was set to 32 amol, 160 amol, 0.8 fmol, 4 fmol, and 20 fmol. As apparent from the results shown in Fig. 20, it was demonstrated that the fluorescence signal increases over time at a reaction velocity dependent on the amount of the target nucleic acid strand.

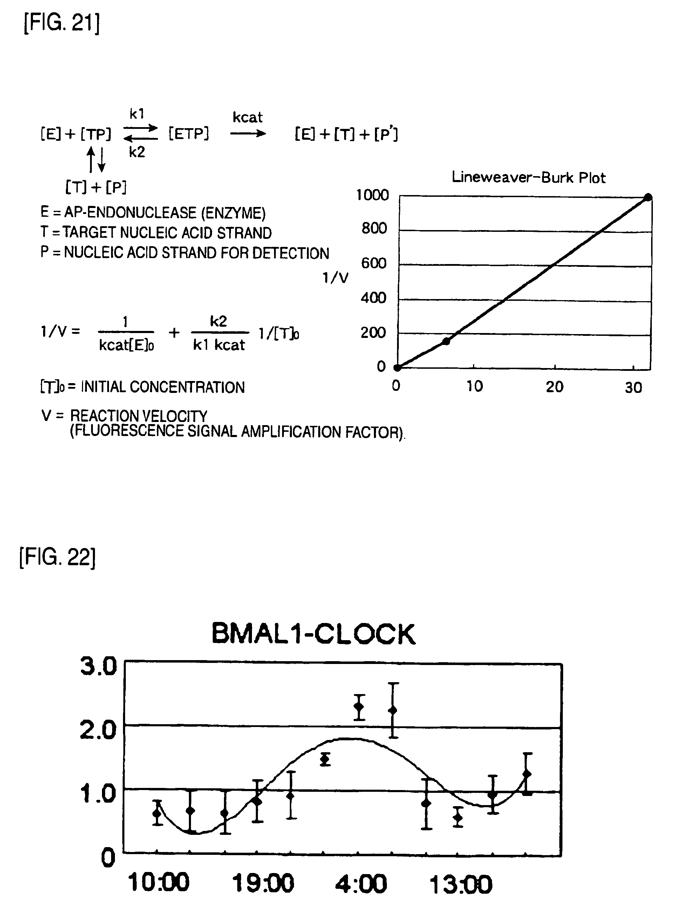

Further, when the respective concentrations of the target nucleic acid strand and an initial reaction velocity were plotted using the "Lineweaver Burk Plot", the relationship is substantially linear in a range from 32 amol to 20 fmol. That is, when the target nucleic acid strand is used as a substrate for the AP-endonuclease, the initial concentration [T]₀ of its template nucleic acid strand and the initial reaction velocity V can be expressed as a linear expression. Accordingly, it was shown that the target nucleic acid strand can be quantitatively determined in the above-mentioned concentration range (see Fig. 21).

### (Experimental example 3)

This experimental example shows that one example of the application examples of the method according to the invention was actually performed and successful. Specifically, this experimental example is an experimental example showing that the detection of a BMAL1-CLOCK complex which is a product of a biological clock gene (clock protein) having a transcription factor activity and also the detection of a diurnal variation rhythm from human oral mucosal epithelial cells were successfully achieved.

Hereinafter, an example of a procedure for detecting the clock protein BMAL1-CLOCK complex from human oral mucosal epithelial cells will be described.

First, a "nucleic acid strand for detection" used in this experimental example is an oligo-DNA having a base sequence structure shown below in which a fluorescent dye (FITC) is bound to the 5' end side and a quencher substance (BHQ) is bound to the 3' end side (the 6th base (guanine) from the 5' end has been deleted: AP site) (see Table 1), and a nucleic acid strand which forms a double strand with this nucleic acid strand for detection is an oligo-DNA having a base sequence structure shown in "Table 2", and as an antibody, anti-BMAL1 antibody (Santa Cruz Biotechnology, Inc.) was used.

**[Table 1]**

| |
|---|
| 5' FITC-acccag (AP) ccacgtgc-BHQ 3' (SEQ ID NO: 4) |

**[Table 2]**

| |
|---|
| 5' gcacgtggatgggt 3' (SEQ ID NO: 5) |

Subsequently, antibody magnetic beads were prepared according to the following procedure. The above-mentioned antibody was conjugated to magnetic beads (micromer-M [PEG-COOH], micromod Partikeltechnologie Gmbh) using PolyLink-Protein Coupling Kit for COOH Microparticles (Polyscience, Inc.).

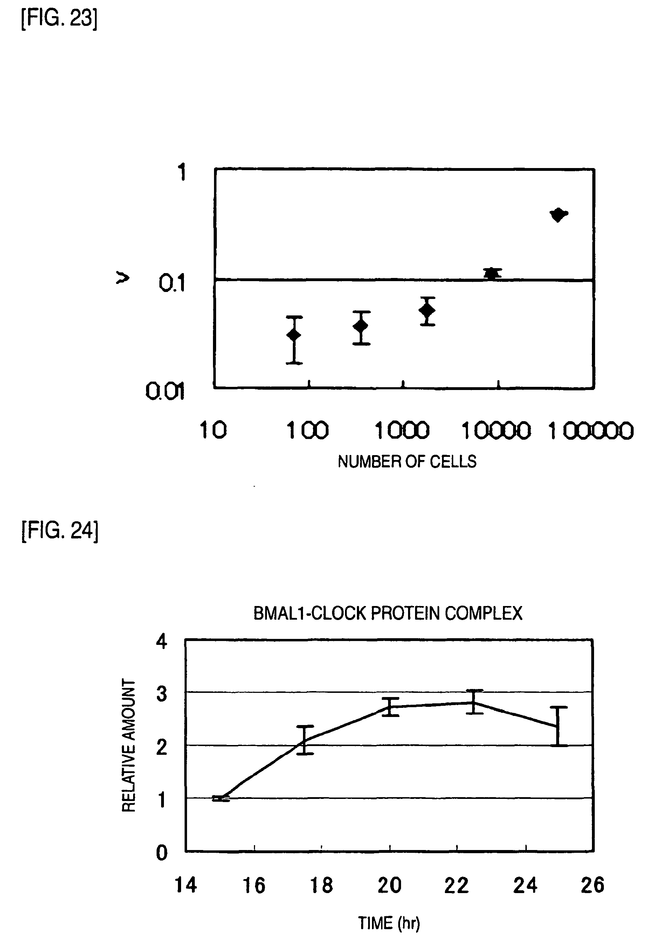

(1) Oral cells collected from a human were suspended in 20 mM Tris-Cl (pH 7.5) containing protease inhibitor cocktail (Roche), 150 mM NaCl, and 1 ucrose monolaurate, and the cells were homogenated using a vortex. (2) The resulting homogenate was centrifuged at 16,000 g for 10 minutes to precipitate insoluble components. (3) Absorbance measurement at 280 nm was performed for the supernatant of the sample obtained in (2), and by using the total protein amount of each of the samples as an index, the protein amount was made equal in all the samples. (4) Each sample in which the protein amount was made equal in the previous procedure was diluted with nine times volume of PBS-T (PBS (pH7.5), 0.05% Tween 20) containing protease inhibitor cocktail. (5) 2 µL of the antibody magnetic beads (anti-BMAL1) were added to each diluted solution and incubated at 4°C for one whole day and night. (6) The antibody magnetic beads were washed with 500 µL of PBS-T to wash away non-specifically bound molecules. (7) Subsequently, the antibody magnetic beads were suspended in 10 mM Tris-Cl (pH 7.5), 50 mM KCl, 2.5 2.27926e+289lycerol, 10 mM EDTA, 0.05 P-40, 0.05 mg/mL salmon sperm DNA, and 0.2 µM each nucleic acid probe for detection (a double-stranded DNA composed of probe nucleic acids 1 and 2), and the resulting suspension was incubated at 37°C for 1 hour. (8) The antibody magnetic beads were washed with 500 µL of PBS-T three times to wash away non-specifically bound molecules and the probe nucleic acids. (9) The antibody magnetic beads were suspended in water and the resulting suspension was incubated at 80°C to extract the specifically bound nucleic acid strand for detection. (10) The nucleic acid strand for detection extracted by the previous procedure was suspended in 20 mM Tris-Acetat, 10 mM Mg-Acetat, 50 mM KCl, 1 mM DTT (pH 7.9) (the final concentrations are shown), and a final concentration of 0.2 µM each of a nucleic acid strand for detection (SEQ ID NO: 4) and an AP-endonuclease were added to the suspension, and the resulting mixture was incubated at 37°C. Then, an increase in fluorescence of the fluorescent dye (FITC) was measured over time. (11) Increasing ratios of fluorescence per unit time against each time point were plotted (see the graph substituted for drawing in Fig. 22). Further, in order to examine the sensitivity, detection of the BMAL1-CLOCK complex was performed according to the same method as described above by changing the number of Hela cells, which was successful. The results are shown in a graph (see the graph substituted for drawing in Fig. 23).

Discussion

A so-called clock protein is a transcription factor, and an autonomous oscillator changing a circadian rhythm which is most important among biological rhythms. It is believed at present that a clock protein being a transcription factor induces the expression of another clock protein, and the induced clock protein functions also as a transcription factor, and functions as a circadian rhythm oscillator by a negative field loop of such as inhibiting the transcription of the other (original) clock protein. That is, the clock protein being a transcription factor is the core of a biological clock, and thus, it is considered that the measurement of such a clock protein is most suitable for the measurement of a biological rhythm of living organisms.

Recently, it has gradually been revealed that defect or diversity in function or gene of a biomolecule involved in a biological clock are causative factors of lifestyle-related diseases such as cancer, diabetes, vascular diseases, and neurodegenerative diseases. In particular, psychiatric disorders such as bipolar disorder and depression are suspected to be caused by an abnormal biological clock. In fact, a therapeutic effect is exhibited by light irradiation which has an effect of resetting the biological clock.

Further, the human sleep-wake cycle is not only autonomously controlled by a biological clock, but also restricted by social life. Therefore, a lag is generated between the rhythm based on the sleep-wake cycle and the autonomous rhythm based on the biological clock, which may cause physical deconditioning as typified by jet lag, and moreover, poor health status as described above. Further, an amount of an enzyme for metabolizing a drug in the body has a circadian rhythm, and also an amount of a molecule to be a target for a drug has a circadian rhythm in many cases. Therefore, it is known that the sensitivity to a drug also has a circadian rhythm, and an idea of time therapy which is performed by setting the time of drug administration is also being spread.

To cite a familiar example, it is known that the mind and body activity and exercise performance also have a circadian rhythm, and a rhythm which maximizes one's own ability, a rhythm which is good for learning or training, an eating rhythm which makes the body weight to increase easily, and the like are considered.

As described above, by applying the nucleic acid strand for detection or the method according to the invention, a clock protein which is one example of transcription factors can actually be measured. From this, it is expected that knowing of the autonomous biological rhythm based on the biological clock or the sleep-wake cycle and a lag thereof with the external time will be a very useful technique for prevention of psychiatric disorders and lifestyle-related diseases, improvement of physical deconditioning such as jet lag, time therapy, exhibition of one's own ability, learning, training, diet, and the like. In addition, as an application example of the invention, the following Experimental example 4 will be described.

### (Experimental example 4)

At present, it is known that by stimulating cultured cells in a culture medium containing 50 horse serum, the expression of clock genes is synchronized among the respective cells, and as a variation of the mRNA levels of the respective clock genes, a rhythm is observed. However, because it was difficult to detect a clock protein so far, a detailed observation of the "expression variation" of a BMAL1-CLOCK protein complex was not made.

Accordingly, by applying the invention, a given experiment for verifying that it is possible to observe the expression variation of a BMAL1-CLOCK protein complex from Hela cells was carried out. Further, variation patterns of mRNAs of genes Per1 and Per2 which contains an E-box sequence to which a BMAL1-CLOCK protein complex is bound in a promoter region and whose transcription is activated by the BMAL1-CLOCK protein complex were observed and compared with the expression variation of the BMAL1-CLOCK protein complex.

Specifically, Hela cells were stimulated in a culture medium (DMEM culture medium) containing 50 horse serum for 2 hours. The cells were recovered at 15 hours, 17.5 hours, 20 hours, and 22.5 hours after initiation of the stimulation, and the BMAL1-CLOCK protein complex was detected according to the same procedure as that for the oral mucosal epithelial cells (see the graph substituted for drawing in Fig. 24).

In addition, the cells were recovered at 12 hours, 16 hours, 20 hours, 24 hours, and 28 hours after initiation of the stimulation. From the respective recovered cells, total RNA was extracted (manufacture name: Agilent Technologies, model No: 5185-6000), PT-PCR was performed according to a conventional method, and then, Per1 and Per2 mRNA levels were quantitatively determined. The sequences of primers used in RT-PCR are as shown in "Table 3".

**[Table 3]**

| | | | |
|---|---|---|---|
| GAPDH | forward | 5' gcaccgtcaaggctgagaac 3' | SEQ ID NO: 6 |
| | reverse | 5' atggtggtgaagacgccagt 3' | SEQ ID NO: 7 |
| Per1 | forward | 5' acgcactggcctgtgtcaag 3' | SEQ ID NO: 8 |
| | reverse | 5' tagacgattcggcccgtca 3' | SEQ ID NO: 9 |
| Per2 | forward | | SEQ ID NO: 10 |
| | reverse | 5' agtaaaagaatctgctccactg 3' | SEQ ID NO: 11 |

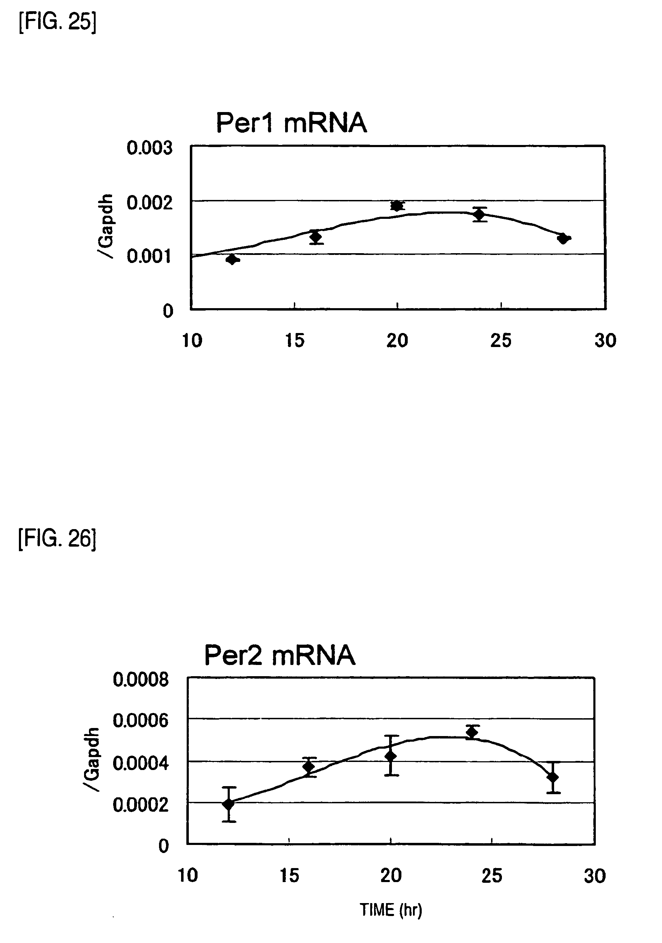

Fig. 25 is a view (graph substituted for drawing) showing results obtained by quantitatively determining the Per1 mRNA level, and Fig. 26 is a view (graph substituted for drawing) showing results obtained by quantitatively determining the Per2 mRNA level. The Per1 and Per2 mRNA levels were calculated as relative values to the level of GAPDH (glyceraldehyde-3-phosphate dehydrogenase) as an endogenous control.

Discussion

Because the variation pattern of the BMAL1-CLOCK protein complex level is similar to the variation patterns of mRNAs of Per1 and Per2 whose transcription is activated by the BMAL1-CLOCK protein complex (see and compare Fig. 24 with Figs. 25 and 26), it was shown that the BMAL1-CLOCK protein complex level reflects the transcriptional activity of the BMAL1-CLOCK protein complex. From these results, it was demonstrated that by applying the invention, a clock protein can be detected, and also a detailed observation of the "expression variation" of the BMAL1-CLOCK protein complex can be achieved.

The experimental examples described above are for illustrating part of application examples of the invention. In the case where transcriptional regulation is achieved by the level of a transcription factor, the degree of modification after translation, or the formation of a complex with another molecule, a cell homogenate liquid can be used as shown in Examples. In the case where transcriptional regulation is independent of the above-mentioned regulatory mechanisms and achieved by nuclear import of a transcription factor, by using a nuclear extract liquid, the degree of activity of a transcription factor can be measured. Further, as for such a transcription factor that is produced as a membrane-associated protein like Notch or SREBP and is cleaved by a protein cleaving enzyme and migrates into the nucleus, the degree of activity of a transcription factor can be measured by using a cell extract liquid without containing a membrane fraction or the like.

In transcription factors, there are a lot of nuclear receptors which do not show a transcriptional activity until they bind to a steroid hormone. By applying the invention, it is also possible to search or quantitatively determine a ligand (such as a steroid hormone) for these nuclear receptors. Further, it is also possible to search, quantitatively determine, or evaluate a drug substance which works as an agonist or an antagonist for such a nuclear receptor. It is also considered that if SREBP which is activated by cholesterol is measured, metabolic syndrome, Alzheimer's disease, or the like can be prevented. Further, by measuring a transcription factor to be a nuclear receptor for a vitamin or a hormone, a physiological condition such as a nutritional condition can be known. When a purified nuclear receptor is used, quantitative determination of a vitamin or a hormone serving as a ligand or measurement of affinity for a nuclear receptor can be achieved, and also the invention can be applied to screening or evaluation of an agonist or an antagonist targeting a nuclear receptor.

### Industrial Applicability

The invention can be used as a technique for fluorescently detecting an infinitesimal substance present in a reaction field with a high sensitivity. For example, it is particularly useful in the (1) detection of a target nucleic acid strand complementary to a nucleic acid strand for detection; (2) detection of a biological substance other than nucleic acids; (3) detection of a chemical change (chemical modification) in a biological substance; (4) detection of a structural change in a protein or other biological substances; (5) detection or screening of a drug candidate substance; (6) detection of an environmental hormone (endocrine disruptor); (7) diagnosis of diseases; and (8) area of a sensor chip such as a DNA chip.

## Claims

1. A nucleic acid strand for detection comprising:
a fluorescent substance which can serve as a donor of resonance excitation energy (fluorescence resonance energy);
a quencher substance which is located at a position enabling it to receive the resonance excitation energy; and
a nucleic acid strand region which is located between the quencher substance and the fluorescent substance and has an enzyme cleavage site, which is an abasic site (AP site) to be cleaved by a double strand-specific AP-endonuclease, wherein in the nucleic acid strand region, a responsive element region which binds to a transcription factor is present, and wherein the nucleic acid strand region is an oligonucleotide strand.

2. A method using a nucleic acid strand comprising at least the steps of:
allowing a complementary strand formation reaction involving a nucleic acid strand for detection according to claim 1; and
cleaving a probe nucleic acid strand at the AP-site by allowing the double strand-specific AP-enconuclease specific for a double strand obtained by the complementary strand formation reaction to act thereon to effect fragmentation and also dissociating the cleaved strand into single strands thereby amplifying the fluorescence of the fluorescent substance.

3. The method using the method according to claim 2, **characterized by** measuring a fluorescence intensity before and after the endonuclease is allowed to act on and detecting an environmental hormone.

4. The method according to claim 3, **characterized in that** the method is performed under a condition of a temperature not higher than an upper limit temperature at which the complementary strand formation reaction of the nucleic acid strand for detection before the cleavage by the endonuclease can be allowed to proceed and not lower than a temperature at which the complementary strand formation reaction of the fragments of the nucleic acid strand for detection after the cleavage by the endonuclease cannot be maintained or allowed to proceed.

5. Use of the nucleic acid strand according to claim 1 for detecting or screening an environmental hormone.

## Patentansprüche

1. Nukleinsäurestrang zum Nachweisen, umfassend:
einen fluoreszierenden Stoff, der als Donor von Resonanzanregungsenergie (Fluoreszenzresonanzenergie) dienen kann;
einen Quencher-Stoff, der sich an einer Position befindet, die es ihm ermöglicht, die Resonanzanregungsenergie zu empfangen; und
einen Nukleinsäurestrangbereich, der zwischen dem Quencher-Stoff und dem fluoreszierenden Stoff liegt und eine Enzymspaltstelle aufweist, bei der es sich um eine von einer doppelstrangspezifischen AP-Endonuklease zu spaltende abasische Stelle (AP-Stelle) handelt, wobei in dem Nukleinsäurestrangbereich ein Responsive-Element-Bereich vorhanden ist, der an einen Transkriptionsfaktor bindet, und wobei es sich bei dem Nukleinsäurestrangbereich um einen Oligonukleotidstrang handelt.

2. Verfahren unter Verwendung eines Nukleinsäurestrangs, umfassend wenigstens die folgenden Schritte:
Gestatten einer Komplementärer-Strang-Bildungsreaktion unter Beteiligung eines Nukleinsäurestrangs zum Nachweisen gemäß Anspruch 1; und Spalten eines Sonden-Nukleinsäurestrangs an der AP-Stelle, indem man die für einen durch die Komplementärer-Strang-Bildungsreaktion erhaltenen Doppelstrang spezifische doppelstrangspezifische AP-Endonuklease darauf wirken lässt, so dass eine Fragmentierung bewirkt wird, und auch Dissoziieren des gespaltenen Strangs in Einzelstränge, wodurch die Fluoreszenz des fluoreszierenden Stoffs amplifiziert wird.

3. Verfahren unter Verwendung des Verfahrens nach Anspruch 2, **gekennzeichnet durch** Messen einer Fluoreszenzintensität, bevor und nachdem man die Endonuklease wirken lässt, und Nachweisen eines Umwelthormons.

4. Verfahren nach Anspruch 3, **dadurch gekennzeichnet, dass** das Verfahren unter einer Bedingung einer Temperatur, die nicht höher als eine obere Grenztemperatur, bei der die Komplementärer-Strang-Bildungsreaktion des Nukleinsäurestrangs zum Nachweisen vor der Spaltung durch die Endonuklease ablaufen gelassen werden kann, und nicht niedriger als eine Temperatur, bei der die Komplementärer-Strang-Bildungsreaktion der Fragmente des Nukleinsäurestrangs zum Nachweisen nach der Spaltung durch die Endonuklease nicht aufrechterhalten oder ablaufen gelassen werden kann, ist, durchgeführt wird.

5. Verwendung des Nukleinsäurestrangs nach Anspruch 1 zum Nachweis oder zum Screening eines Umwelthormons.

## Revendications

1. Brin d'acide nucléique pour détection, comprenant:
une substance fluorescente qui peut servir de donneur d'énergie d'excitation de résonance (énergie de résonance par fluorescence) ;
une substance extinctrice, qui est située sur une position lui permettant de recevoir l'énergie d'excitation de résonance ; et
une région du brin d'acide nucléique qui est située entre la substance extinctrice et la substance fluorescente, et possède un site de clivage enzymatique, qui est un site abasique (site AP) devant être clivé par une AP-endonucléase spécifique de double brin, où, dans la région du brin d'acide nucléique, une région élément sensible, qui se lie à un facteur de transcription, est présente, et où la région du brin d'acide nucléique est un brin oligonucléotidique.

2. Procédé utilisant un brin d'acide nucléique, comprenant au moins les étapes consistant :
à permettre une réaction de formation d'un brin complémentaire impliquant un brin d'acide nucléique pour détection selon la revendication 1 ; et
à cliver un brin d'acide nucléique sonde au niveau du site AP, en permettant à l'AP-endonucléase spécifique de double brin, spécifique d'un double brin obtenu par la réaction de formation d'un brin complémentaire, d'agir sur ce dernier pour provoquer une fragmentation et aussi une dissociation du brin clivé en des brins individuels, ce qui va amplifier la fluorescence de la substance fluorescente.

3. Procédé utilisant le procédé selon la revendication 2, **caractérisé par** la mesure d'une intensité de fluorescence avant et après que l'on a permis à l'endonucléase d'agir, et la détection d'une hormone environnementale.

4. Procédé selon la revendication 3, **caractérisé en ce que** le procédé est mis en oeuvre dans des conditions de température non supérieure à une température limite supérieure à laquelle la réaction de formation du brin complémentaire du brin d'acide nucléique pour la détection avant le clivage par l'endonucléase puisse être amenée à se réaliser, et non inférieure à une température à laquelle la réaction de formation du brin complémentaire des fragments du brin d'acide nucléique pour détection après le clivage par l'endonucléase ne puisse être maintenue, ou être amenée à se réaliser.

5. Utilisation d'un brin d'acide nucléique selon la revendication 1 pour détecter ou cribler une hormone environnementale.


### SEQUENCE LISTING

<110>Sony Corporation
<120> Nucleic acid chain for detecting material and the method by using the
   nucleic acid chain
<130>0790375302
<160>3
<210>1
   <211>17
   <212>DNA
   <213>Artificial sequence
<220>
   <223>Target nucleotic acid chain
<400>1
   agcacggctt ctcctta 17
<210>2
   <211>17
   <212>DNA
   <213>Artificial sequence
<220>
   <223>Probe nucleotic acid chain
   n means Apurinic/Apyrimidinic site
<400>2
   taaggagaan ccgtgct
<210>3
   <211>318
   <212>PRT
   <213>Homo sapiens
<220>
   <223>human AP-endonuclease(APEl)
<400>3
   Met Pro Lys Arg Gly Lys Lys Gly Ala Val Ala Glu Asp Gly Asp
   1               5                   10                  15
   Glu Leu Arg Thr Glu Pro Glu Ala Lys Lys Ser Lys Thr Ala Ala
                   20                  25                  30
   Lys Lys Asn Asp Lys Glu Ala Ala Gly Glu Gly Pro Ala Leu Tyr
                   35                  40                  45
   Glu Asp Pro Pro Asp Gln Lys Thr Ser Pro Ser Gly Lys Pro Ala
                   50                  55                  60
   Thr Leu Lys Ile Cys Ser Trp Asn Val Asp Gly Leu Arg Ala Trp
                   65                  70                  75
   Ile Lys Lys Lys Gly Leu Asp Trp Val Lys Glu Glu Ala Pro Asp
                   80                  85                  90
   Ile Leu Cys Leu Gln Glu Thr Lys Cys Ser Glu Asn Lys Leu Pro
                   95                  100                 105
   Ala Glu Leu Gln Glu Leu Pro Gly Leu Ser His Gln Tyr Trp Ser
                   110                 115                 120
   Ala Pro Ser Asp Lys Glu Gly Tyr Ser Gly Val Gly Leu Leu Ser
                   125                 130                 135
   Arg Gln Cys Pro Leu Lys Val Ser Tyr Gly Ile Gly Asp Glu Glu
                   140                 145                 150
   His Asp Gln Glu Gly Arg Val Ile Val Ala Glu Phe Asp Ser Phe
                   155                 160                 165
   Val Leu Val Thr Ala Tyr Val Pro Asn Ala Gly Arg Gly Leu Val
                   170                 175                 180
   Arg Leu Glu Tyr Arg Gln Arg Trp Asp Glu Ala Phe Arg Lys Phe
                   185                 190                 195
   Leu Lys Gly Leu Ala Ser Arg Lys Pro Leu Val Leu Cys Gly Asp
                   200                 205                 210
   Leu Asn Val Ala His Glu Glu Ile Asp Leu Arg Asn Pro Lys Gly
                   215                 220                 225
   Asn Lys Lys Asn Ala Gly Phe Thr Pro Gln Glu Arg Gln Gly Phe
                   230                 235                 240
   Gly Glu Leu Leu Gln Ala Val Pro Leu Ala Asp Ser Phe Arg His
                   245                 250                 255
   Leu Tyr Pro Asn Thr Pro Tyr Ala Tyr Thr Phe Trp Thr Tyr Met
                   260                 265                 270
   Met Asn Ala Arg Ser Lys Asn Val Gly Trp Arg Leu Asp Tyr Phe
                   275                 280                 285
   Leu Leu Ser His Ser Leu Leu Pro Ala Leu Cys Asp Ser Lys Ile
                   290                 295                 300
   Arg Ser Lys Ala Leu Gly Ser Asp His Cys Pro Ile Thr Leu Tyr
                   305                 310                 315
   Leu Ala Leu
           318
<210>4
   <211>15
   <212>DNA
   <213>Artificial sequence
<220>
   <223>n means Apurinic/Apyrimidinic site
<400>4
   acccagncca cgtgc 15
<210>5
   <211>15
   <212>DNA
   <213>Artificial sequence
<220>
   <223>
<400>5
   gcacgtggac tgggt 15
<210>5
   <211>15
   <212>DNA
   <213>Artificial sequence
<220>
   <223>
<400>5
   gcacgtggac tgggt 15
<210>6
   <211>20
   <212>DNA
   <213>Artificial sequence
<220>
   <223>forward primer for RT-PCR
<400>6
   gcaccgtcaa ggctgagaac 20
<210>7
   <211>20
   <212>DNA
   <213>Artificial sequence
<220>
   <223>reverse primer for RT-PCR
<400>7
   atggtggtga agacgccagt 20
<210>8
   <211>20
   <212>DNA
   <213>Artificial sequence
<220>
   <223>forward primer for RT-PCR
<400>8
   acgcactggc ctgtgtcaag 20
<210>9
   <211>19
   <212>DNA
   <213>Artificial sequence
<220>
   <223>reverse primer for RT-PCR
<400>9
   tagacgattc ggcccgtca 19
<210>10
   <211>24
   <212>DNA
   <213>Artificial sequence
<220>
   <223>forward primer for RT-PCR
<400>10
   gcatccatat ttcactgtaa aaga 24
<210>11
   <211>22
   <212>DNA
   <213>Artificial sequence
<220>
   <223>reverse primer for RT-PCR
<400>11
   agtaaaagaa tctgctccac tg 22